(19) **Europäisches Patentamt / European Patent Office / Office européen des brevets**

(11) **EP 3 984 961 A1**

(12) **EUROPEAN PATENT APPLICATION**

(43) Date of publication:
**20.04.2022 Bulletin 2022/16**

(21) Application number: **20202041.8**

(22) Date of filing: **15.10.2020**

(51) International Patent Classification (IPC):
**C02F 1/32** $^{(2006.01)}$ **A61L 2/10** $^{(2006.01)}$

(52) Cooperative Patent Classification (CPC):
**C02F 1/325; A61L 2/10;** A61L 2202/11;
A61L 2202/14; C02F 2201/3222; C02F 2201/3227;
C02F 2201/326

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Grundfos Holding A/S**
**8850 Bjerringbro (DK)**

(72) Inventors:
• **Højris, Bo**
  **8850 Bjerringbro (DK)**
• **D'Antonio, Sébastien**
  **8850 Bjerringbro (DK)**
• **Smith, Christian Guldbæk**
  **8850 Bjerringbro (DK)**
• **Larsen, Claus Bo**
  **8850 Bjerringbro (DK)**

(74) Representative: **Plougmann Vingtoft a/s**
**Strandvejen 70**
**2900 Hellerup (DK)**

(54) **A METHOD AND REACTOR FOR EXPOSING A FLUID TO UV RADIATION**

(57) The present invention relates to a method for determining quality of a fluid, preferably being water. The method involves exposing the fluid to UV radiation to determine a parameter, such as absorbance of the fluid, related to the quality of the fluid and/or for disinfecting the fluid. The method may also involve determining a fouling factor. The invention also relates to a reactor adapted to expose a fluid to UV radiation, the reactor comprising an UV arrangement comprising at least two UV sources and at least one UV sensor. The two UV sources and the one UV sensor being positioned relatively to each other so that the distances ($d_1$, $d_2$) from UV sensor and to each of the UV sources are different from each other so as to provide two different optical path lengths.

Fig. 1

EP 3 984 961 A1

**Description**

FIELD OF THE INVENTION

The present invention relates to a method for determining quality of a fluid, preferably being water. The method involves exposing the fluid to UV radiation to determine a parameter, such as absorbance of the fluid, related to the quality of the fluid and/or for disinfecting the fluid. The method may also involve determining a fouling factor. The invention also relates to a reactor adapted to expose a fluid to UV radiation, the reactor comprising an UV arrangement comprising at least two UV sources and at least one UV sensor. The two UV sources and the one UV sensor being positioned relatively to each other so that the distances ($d_1$, $d_2$) from UV sensor and to each of the UV sources are different from each other so as to provide two different optical path lengths.

BACKGROUND OF THE INVENTION

Broadly, the technical application of monitoring water quality applications can be divided into the following areas: organic content, microbes, heavy metals and inorganic content.

UV disinfection of water is a process typically used when chemicals like chlorine are undesirable. Monitoring of water quality is typically done when either legislation require it, or when processes are to be optimized. It may therefore be desired to be able to address the organic content of water by measuring the UV transmission of water in real time.

Having the ability to control a UV disinfection based on information about the water quality is found to be a treasured aim. However, sensors used for determining e.g. absorbance of water in a UV disinfection reactor are expensive and requires special fittings.

In traditional UV reactors, a UV sensor is sometimes installed, typically because it is required by legislation. This sensor is installed so that it receive light from the mercury lamps, and its output is used as an indicator to show that the reactor is working properly. With this approach, the sum of light source degradation, fouling and changes in UV transmittance is tracked, but it is impossible to say which of the three scenarios is causing the reduced light.

Hence, an improved method and device for determining water quality in relation to UV radiation would be advantageous, and in particular, a more efficient and/or reliable method and device would be advantageous.

OBJECT OF THE INVENTION

It is an object of the invention to at least mitigate the aforementioned problems pertaining to determining water quality and exposing water to UV radiation.

It is a further object of the present invention to provide an alternative to the prior art.

In particular, it may be seen as an object of the present invention to provide a method and device that solves the above mentioned problems of the prior art.

SUMMARY OF THE INVENTION

Thus, the above described object and several other objects are intended to be obtained in a first aspect of the invention by providing a reactor adapted to expose a fluid to UV radiation, the reactor comprising

- a container having a void for holding a fluid, and
- an UV arrangement comprising at least two UV sources being configured to emit UV light when electrically powered and at least one UV sensor being configured to provide an electrical output related to received UV intensity when exposed to UV light, said two UV sources and said one UV sensor being positioned relatively to each other so that the distances from said UV sensor and to each of said UV sources are different from each other so as to provide two different optical path lengths, said two UV source being individually on-off switchable, and

    ○ the UV sources being arranged in the container to expose at least a part of the void to UV radiation, and
    ○ the UV sensors being arranged in the container to receive light emitted from the UV sources.

It is noted that the invention is not limited to the use of two UV sources and one UV sensor, and more of each may be used in connection with the present invention.

The reactor may preferably be for determining a quality of a fluid, such as water as otherwise disclosed herein. The reactor may preferably further comprising a control unit configured to determine the absorbance based on input from the UV sensor in response to said UV sources emit light through the water and onto the UV sensor.

In an overall perspective, the purpose of exposing fluid, such as water, to UV radiation is to measure and/or control the quality of the water, such as determining the absorbance of the water, which provides an indication of the level of dirtiness e.g. due to presence of bacteria and/or control the level of bacteria by exposing the bacteria to UV radiation thereby at least partly disinfecting the water.

In relation to the present invention, *"water quality"* typically refers to the amount of bacteria that can be killed by UV radiation and where the amount of such bacteria is correlated with the UV light absorbance of the water. In relation to the present invention, the absorbance of the water is determined by use of UV light emitted at one or several wavelengths. This determined absorbance depends on the content and composition of organic matter in the water whereby the absorbance determined is used as an indicator for the quality of the water

The invention resides inter alia in determinations of light emitted and sensed in multiple different optical path lengths. As presented herein, this is preferably obtained by at least two UV sources placed at positions distanced differently from an UV sensor. By the difference in optical path lengths and the ability of turn the light source on and off individually, the present invention provides the advantages of inter alia determining the absorbance essentially independently of the intensity at which the light is emitted as well as being able to determine a measure for the fouling in a reactor.

Terms used herein are used in a manner being ordinary to a skilled person. Some of terms used are detailed here below:

*Cycle* is typically used to mean the process of switching light sources on and off sequentially.

*Operation* is typically used to mean the state in which the light sources are used for disinfection.

*Light source* is typically used to mean a single light emitting element or an assembly of light emitting elements operational in common.

UV as used herein typically refers to the abbreviation of ultra violet. When used in connection with light as UV light source, reference is made to ultra violet light and when used in connection with sensor, reference is made to ultra violet light sensor. Preferably, the invention utilizes UV light with wavelength in UVC band of 200-280 nm, UVA band of 315-400 nm and/or in the UVB band of 280-315 nm.

In some preferred embodiment of a the container may have a tubular section inside which the void may be present. A first opening may preferably be provided at one end of the tubular section and a second opening may be provided at another end of the tubular section. The first and the second openings typically provides passages for fluid to enter and leave the void.

In some preferred embodiments, the container may have a tubular section inside which the void may be present. A first and a second opening may preferably be provided and the first and the second openings may provide passages for fluid to enter and leave said void. The first and second openings may preferably be arranged so that reactor may be configured for in-line connection with a piping arrangement.

The reactor may in some preferred embodiments preferably be used for disinfection a fluid and/or for determining the absorbance of a fluid and/or for determining a fouling factor and/or for other purposes presented herein.

In some preferred embodiment, the invention relates to a system for disinfecting a fluid where the system preferably comprising a pump in fluidic connection with the reactor according the first aspect. In some preferred embodiments, a flow of water through the reactor may be provided by flow of water provided by water works.

In some preferred embodiments of a system for disinfection a fluid, the system may preferably comprising a flow sensor, such as a flow switch, preferably incorporated with the reactor according to the first aspect. The flow sensor preferably being configured to provide an electrical output related to fluid flow into/out from said void.

In a second aspect the invention relates to a method for examining the state of the light sources 4 in a reactor according to the first aspect of the invention, the method comprising a first measuring cycle comprising sequentially switching each light source on and off, or turning one or more light source sequentially off, and recording the light intensity received by the sensor thereby, repeat this first measuring cycle at a later instant and derive from such two measuring cycles decade, if present, of each of the light sources.

In a third aspect, the invention relates to a method for determining absorbance in a fluid exposed to UV radiation in a by use of light emitted and sensed in multiple different optical path lengths, the method is based on an a priori determined correlation between light intensity ($I_0$) emitted by light source(s) (4), the absorbance ($a$) and the light intensity ($I_D$) determined by use of a UV sensor the method comprising

- exposing the fluid to light and determining or estimating the intensity ($I_0$) emitted by the light source(s), and
- recording while exposing the fluid to said light, the light intensity ($I_D$) received by the sensor by use of said UV sensor, and
- determining the absorbance ($a$) based said correlation.

In some preferred embodiments, the light intensity ($I_0$) emitted by the light source(s) may be estimated as based on the power consumption of the light source(s).

In some preferred embodiments, the correlation may preferably also include a fouling factor ($k_f$) to take into account, preferably when determining the absorbance (*a*), that less light may be received by the UV sensor(s) due to fouling.

In some preferred embodiments, the fouling factor may preferably be determined at consecutive points in time, such as at regular intervals.

In some preferred embodiments, the fouling factor may be determined by a method preferably comprising:

- obtaining at least two timewise separate measurements of UV light emitted by each of the UV sources (4), or by turning one or more light source sequentially off, by use of the UV sensor,
- determining based on said two timewise separate measurements an absorption coefficient, or a parameter related to the absorption coefficient, for the fluid through which the UV light has travelled, and
- determining based on the absorption coefficient or the related parameter a measure for fouling, such as a fouling factor, of the light source(s) and the UV sensor(s).

In some preferred embodiments, the method may be carried out on a batch of fluid, and wherein the batch of fluid preferably may be kept unchanged in volume and composition during the during the determinations.

In some preferred embodiments, the method may preferably further comprise comparing measurements of UV light from a light source with previous obtained measurement(s) of UV light from the same light source.

In some preferred embodiments, the timewise separate measurements of UV light emitted by the UV source(s) may preferably be obtained in a cycle where said light sources are sequentially switched off and on.

The invention also relates to a device, such as the reactor as disclosed herein or a system as disclosed herein, wherein the device is configured to carry out the method as disclosed herein.

Methods according to the third aspect are preferably deployed in a reactor according to the first aspect.

BRIEF DESCRIPTION OF THE FIGURES

The present invention and preferred embodiments according to the invention will now be described in more detail with regard to the accompanying figures. The figures show ways of implementing the present invention and are not to be construed as being limiting to other possible embodiments falling within the scope of the attached claim set.

Figure 1 is a schematically illustration of a configuration of UV sensor and UV source according to two embodiments of the invention,

Figure 2 is a cross sectional view of a schematically illustrated reactor according to another preferred embodiment of the invention,

Figure 3 is cross section view of a reactor according to another preferred embodiment of the invention; in figure 3 some of the elements of the reactor are shown magnified,

Figure 4 is front view of a schematically illustration of the light source of the embodiment shown in fig. 3,

Figure 5 is a side view of a pump with a reactor according to another embodiment of the invention,

DETAILED DESCRIPTION OF EMBODIMENTS

Prior to detailing the content of the figures, a description of some of the inventive concept are disclosed.

Kindly observe that the figures giving e.g. for number light sources are for illustration and shall not be interpreted as limiting for the scope of the invention.

In some embodiments, the invention makes use of Lambert Beer's law which can be written as:

$$I_d = I_0 e^{-ad} \qquad (1)$$

Where $I_d$ is the light intensity at a distance d from a light source and *a* is an absorption coefficient of the medium through which the light has travelled.

In relation with the present invention, it has been realized that the light received by a sensor and/or emitted by a light source may be influenced by at least two phenomena, namely fouling on the light source and/or sensor and the geometrical orientation between light source and sensor. The latter may be caused e.g. by the sensor having an angle dependency.

In order to take into account these phenomena, Lambert Beer's law is modified by introducing two factors: $k$ accounting for the geometrical set-up and $k_f$ accounting for fouling. Lambert Beer's law in its modified form is expressed as:

$$I_d = k k_f I_0 e^{-ad} \qquad (2)$$

$k$ is considered to be a constant not changing over time (unless the mutual positioning of the sensor and light source is changed) and $k_f$ (herein labelled fouling factor) is a number representing the actual fouling of the sensor and light source in common. Thus, during use the fouling factor, $k_f$, is most likely to change over time.

In the present invention, (at least) two light sources and one sensor are used. In the following the invention is disclosed with reference to embodiments having (at least) two lights sources and one sensor; although derivations below will be similar.

With a system comprising two light sources and one sensor, the following set of modified Lambert Beers equations can be presented:

$$I_{d_1} = k_1 k_f I_0 e^{-ad_1} \qquad (3)$$

$$I_{d_2} = k_2 k_f I_0 e^{-ad_2} \qquad (4)$$

wherein $d_1$ is the distance from one light source and $d_2$ is the distance to another light source from the sensor. It is noted that the fouling factor $kf$ is considered to be composed by a contribution from fouling of sensor and a contribution from fouling of the light source.

By dividing $I_{d_1}$ with $I_{d_2}$ and based on equation (3) and (4) the absorbance can be determined as:

$$a = \frac{ln\left(\frac{I_{d_1}k_2}{I_{d_2}k_1}\right)}{d_2 - d_1} \qquad (5)$$

Examining this equation shows that the absorbance can be determined independently of the fouling of the light sources and/or sensor as the fouling factor $kf$ has been eliminated. Further, the absorbance can be determined independently of $I_0$.

In case with two light sources one is faced with the fact that the sensor accumulates the contribution from both light sources and one cannot determine the ratio between $I_{d1}$ and $I_{d2}$ since the light received by the sensor is composed by light emitted from both sources. This problem has been solved in the present invention by introducing cycle involving a special measuring sequence in which the light sources sequentially are switched on-off so than only one light source at a time is emitting light.

The cycle can be carried out by first switching a first light source on and recording $I_{d1}$ where after the first light source is switch off. When the first light source has been switch off, a second light source is switch on and $I_{d2}$ is recorded. A standstill period between switching off the first light source and switching on the second light source can be introduced in order make sure that no light is emitted from the first light source when the second one is switch on. This may be relevant in case switching off a light source result in a gradually dimming of the light. In embodiments using LEDs this may not be highly relevant, since LEDs essentially stops emitting light when power is shut-off. However, it may be relevant to consider the characteristic of the power supply in the sense that it may be preferred that the switching on and off the power is substantially a step-function.

While the above equation (5) can be used to determine the absorbance of a fluid during operation where disinfection of fluid takes place, it may be preferred not to switch off light sources during the operation since the disinfection could be less effective when some of the light sources are switched off. Further, an aim of the invention is to be able to determine whether fouling has occurred on lights sources and/or sensor. To take into account these boundary conditions, the following is applied.

Assuming that the system when initially put in operation, e.g. following a production process or a cleaning process, has no fouling. This translates into having a fouling factor equal to 1.0, that is $k_f$=1.0. By this, equation (3) above is reduced to:

$$I_{d_1} = k_1 I_0 e^{-ad_1} \qquad (6)$$

A new constant is introduced as:

$$k_{start_1} = k_1 I_0 = \frac{I_{d_1}}{e^{-ad_1}} \qquad (7)$$

This constant, $k_{start1}$, can be determined during a cycle executed when the reactor is clean (or when at least the light sources and sensor are free of fouling) as it depends on geometry $k_1$ and $I_0$. Alternatively, the absorbance, $a$, can be determined by equation (5). Introducing this constant into equation (3) and rearranging to isolate $k_f$ gives the following:

$$k_f = \frac{I_{d_1}}{k_{start_1} e^{-ad_1}} \qquad (8)$$

It is worth noting that $I_{d_1}$ is the number determined during the cycle of sequential switching off and on of the light sources prior to operation, as disclosed above, whereby equation (8) may be re-written as:

$$k_f = \frac{c}{e^{-ad_1}} \qquad (8')$$

where $c$ is a constant.

Thus, with the absorbance, $a$, determined, $k_f$ can be determined by equation (8). By examining the magnitude of the fouling factor $k_f$ it can be realized that fouling has occurred in case $k_f < 1.0$.

As outlined above, the absorbance, $a$, can be determined by equation (5). Use of this equation requires sequential switching off and on of lights sources, and it may be preferred to determine the absorbance in a different manner.

During operation, that is while disinfection takes place, all light sources are typically turned on and emitting light with an intensity of $I_0$. In this case, Lambert Beers equation can be written as

$$I_D = k_D k_f I_0 e^{-aD} \qquad (9)$$

Where capital $D$ refers to an estimate on a distance between the light sources and sensor e.g. calculated as a mean distance from sensor to light sources. Since production typically is initiated right after the cycle of sequential turning light sources on and off, during which cycle $a$ and $k_f$ are determined, $k_D$ can be determined by equation (9). It is noted that since $k_D$ is depending on geometry, it will not change over time and $k_D$ can be determined when the reactor is assembled or after a cleaning process by setting $k_f$ equal to 1.0. $I_0$ is considered known e.g. based on electrical effect supplied to the light sources and $I_D$ is measured by the sensor.

The absorbance, $a$, should preferably be determined during operation. However, while equation (9) finds use during operation, the use of this equation requires knowledge of the fouling factor $k_f$. Thus, if one knew the value of the fouling factor $k_f$ in equation (9) the absorbance can be determined whenever desired.

It has been realized in connection with the present invention, that there are two different characteristic time scale involved in the timewise rate of change of absorbance and fouling. Often the absorbance change more rapidly than the fouling and it has been found that the fouling factor $kf$ may be assumed to be constant for e.g. 24 hours. By determining the fouling factor $k_f$ during a cycle by one of the equations (3), (4) or (8) and using the assumption of the fouling factor being constant during operation, equation (9) can be used to determine the absorbance when desired.

In many practical applications of the invention, the usage of water varies during e.g. 24 hours and during night, the consumption falls to zero during which time there is no need for disinfecting water. During such a period, the fouling factor $k_f$ can be determined in a cycle.

**Summary of preferred method**

*Pre-initial procedure:*

**1.** Determine $k_1$ and $k_2$ of equations (3) and (4). It is noted that these constants are determined based on a liquid representing a batch of water to be disinfected being present in the reactor

*Cycle:*

2. The batch of water (from item 1. Above) is maintained in the reactor

3. Turn sequentially light sources on-off to determine the numerical value of:

$$I_{d_1}; I_{d_2}$$

$$a = \frac{ln\left(\frac{I_{d_1}k_2}{I_{d_2}k_1}\right)}{d_2 - d_1}$$

$$k_{start_1} = \frac{I_{d_1}}{e^{-ad_1}}$$

$$k_f = \frac{I_{d_1}}{k_{start_1}e^{-ad_1}}$$

*Operation*

4. As operation follows the cycle during which focus has not been on disinfection, the batch of fluid used during the cycle is disinfected by turning on all the light sources before the batch is discharged. Thereafter, water to be disinfected flow through the reactor in response to an actual usage of water and exposed to light. Assuming the fouling factor, *kf*, to be constant, the absorbance, *a*, is determined by

$$I_D = k_D k_f I_0 e^{-aD}$$

In a preferred embodiment, the reactor comprises a flow sensor detecting flow of fluid into (or out from) the void of the reactor. In such embodiments, the read-out from the flow sensor is use to turn the light sources on and off, so that the light sources are not turned-on in case there is no flow of fluid through the reactor, or said in another manner, the operation is put in standstill when there is no flow of fluid. However, to assure disinfection the light sources are turned on as fast as possible when a flow is registered by the sensor, and when no flow is registered, the light sources are kept turned on for a sufficient time to assure that the batch of fluid in the void of the reactor is disinfected.

*Observation duty (during disinfection cycle)*

Knowledge about the magnitude of the absorbance can be utilized in a number of ways, such as:

- Determining the level of impurities in the water
- Signalling to a user whether or not the reactor is capable of disinfecting the water (e.g. if absorbance is too high, the UV intensity exposed to the water may not be sufficient)

Knowledge about the fouling can also be used in a number of ways, such as

- Signalling to a user whether or not the reactor is capable of disinfecting the water (e.g. if fouling is too high, the UV intensity exposed to the water may not be sufficient)
- Setting an alarm signalling that service is required

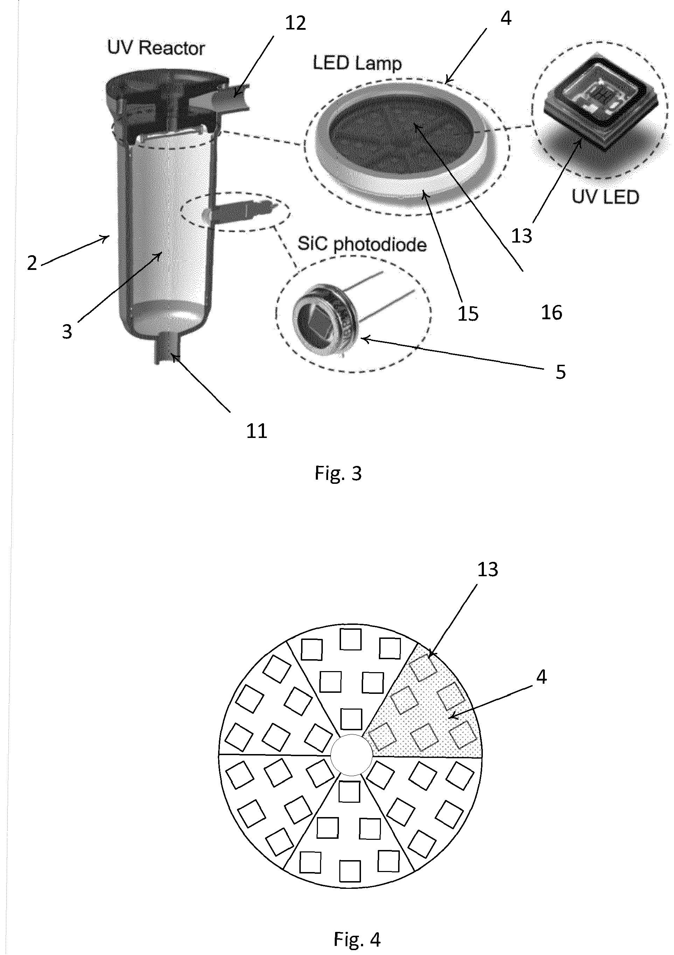

While the above has focussed on turning light sources on and off sequentially, the invention is not limited to such sequentially turning on and off. In many embodiments, a plurality of light sources are applied such as six as shown in fig. 3. Instead of turning the light sources sequentially on and off, it is sufficient to turn one of the light sources off at a time. This has the advantage that there may often still be sufficient light radiated into the water to provide a disinfection, whereby the need for at specific designed cycle may be omitted.

The procedure is detailed in the following with respect to Lambert-Beers law and with reference to a illustrative example where 6 lights sources are used.

The intensity at sensor with 6 light sources on:

$$I_{d_{123456}} = \sum_{n=1}^{6} I_{d_n}$$

Kindly note the sum notation used in symbolic in the sense that one does not mathematically sum up six independent measurement, the sum sign is used to indicate the total light from the light sources considered. Similarly a summation like

$$I_{d_{13456}} = \sum_{n=1,3}^{6} I_{d_n}$$

Is used to refer to the total light from the light sources #1, #3, #4, #5 and #6 on and #2 off.

Intensity at sensor with 5 light sources on and with a first light source (e.g. #1) off:

$$I_{d_{23456}} = \sum_{n=2}^{6} I_{d_n}$$

Intensity at sensor with 5 light sources on with a second light source (e.g. #2) off:

$$I_{d_{13456}} = \sum_{n=1,3}^{6} I_{d_n}$$

Difference in intensity at sensor between 5 light sources and 6 turned on (light source #1 turned off):

$$I_{d_1} = \sum_{n=1}^{6} I_{d_n} - \sum_{n=2}^{6} I_{d_n}$$

Similarly for $I_{d2}$:

$$I_{d_2} = \sum_{n=1}^{6} I_{d_n} - \sum_{n=1,3}^{6} I_{d_n}$$

Thus $I_{d_1}$ $I_{d_2}$ is determined based on all light sources turned, and turning sequentially one light source of two off.

Considering Lambert-Beers law as composed by superimposed contributions from each light source, this law can be written as:

$$I_{d_{123456}} = \sum_{n=1}^{6} I_{d_n} = \sum_{n=1}^{6} k_n k_f I_{0_n} e^{-a d_n}$$

Each contribution can be considered independently, such as $I_{d_1}$, $I_{d_2}$ the following set of equations is available.

$$I_{d_1} = k_1 k_f I_{0_1} e^{-ad_1}$$

$$I_{d_2} = k_2 k_f I_{0_2} e^{-ad_2}$$

Assuming that $I_{d_1} = I_{d_2}$ the absorbance may be eliminated from the above equations as:

$$a = \frac{ln\left(\frac{I_{d_1}k_2}{I_{d_2}k_1}\right)}{d_2 - d_1}$$

Thus, by use of three measurement i) all lights turned on, ii) one first light turned Intensity at sensor with 6 light sources on:

$$I_{d_{123456}} = \sum_{n=1}^{6} I_{d_n}$$

Intensity at sensor with 5 light sources on with a first light source (e.g. #1) off:

$$I_{d_{23456}} = \sum_{n=2}^{6} I_{d_n}$$

Intensity at sensor with 5 light sources on with a second light source (e.g. #2) off:

$$I_{d_{13456}} = \sum_{n=1,3}^{6} I_{d_n}$$

Difference in intensity at sensor between 5 light sources and 6 turned on (light source #1 turned off):

$$I_{d_1} = \sum_{n=1}^{6} I_{d_n} - \sum_{n=2}^{6} I_{d_n}$$

Similarly for $I_{d2}$:

$$I_{d_2} = \sum_{n=1}^{6} I_{d_n} - \sum_{n=1,3}^{6} I_{d_n}$$

Thus $I_{d_1}$ $I_{d_2}$ is determined based on all light sources turned, and turning sequentially one light source of two off.
Considering Lambert-Beers law as composed by superimposed contributions from each light source, this modified law can be written as:

$$I_{d_{123456}} = \sum_{n=1}^{6} I_{d_n} = \sum_{n=1}^{6} k_n k_f I_{0_n} e^{-ad_n}$$

Each contribution can be considered independently, such as $I_{d_1}$ $I_{d_2}$ the following set of equations is available.

$$I_{d_1=}k_1 k_f I_{0_1} e^{-ad_1}$$

$$I_{d_2=}k_2 k_f I_{0_2} e^{-ad_2}$$

Assuming that $I_{0_1} = I_{0_2}$ the absorbance may be eliminated from the above equations as:

$$a = \frac{ln\left(\frac{I_{d_1} k_2}{I_{d_2} k_1}\right)}{d_2 - d_1}$$

Thus, by use of three measurement i) all lights turned on, ii) one first light turned off, and iii) one second light turned, the absorbance can be determined.

The above has also be focussed towards embodiments in which the light emitted during cycle, production and observation is emitted with the same wavelength. However, the invention is not limited to such embodiments, as the light emitted during a cycle may be emitted at wavelength being different from the light emitted during the production and/or operation. In such situations, different light sources are typically involved, since e.g. an LEDs emit light with a quite narrow wavelength span.

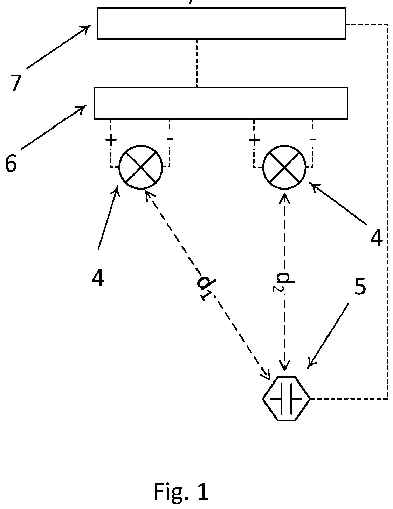

Reference is now made to fig. 1 schematically illustrating an UV arrangement according to preferred embodiments of the invention. The UV arrangement comprises UV sources 4 being configured to emit UV light when electrically powered and one or more UV sensors 5 being configured to provide an electrical output related to received UV intensity when exposed to UV light. The UV sources 4 and UV sensor(s) 5 are preferably standard components readily available.

With reference to fig. 1 an embodiment of UV arrangement is illustrated. In the embodiment the UV arrangement has at least two UV sources 4 and a UV sensor 5. It is noted that more light sources and sensors may be included, however the two UV sources 4 and one UV sensor 5 shown are those involved in the above disclosed use of Lambert-Beers law. The two UV sources 4 and said one UV sensor 5 are spatially positioned relatively to each other so that the distances $d_1$, $d_2$ from said UV sensor 5 and to each of said UV sources 4 are different from each other, that is the distance $d_1$ is different from $d_2$. The distances considered are the direct distance from the source 4 and the sensor 5. In situations where the light source 4 and/or the sensor 5 has/have a substantial geometrical span, the distance is typically measured from a geometrical midpoint. The two UV sources 4 being individually on-off switchable, that is one of the UV sources can be switching on and off without switching the other one on and off (and vice versa)

As also illustrated in fig. 1, the UV arrangement has a power supply 6 electrical connected to said light source(s) to provide electrical power to said light source(s) 4. The power supply 6 typically convert main into a power signal appropriate for the light source(s) 4. While the power supply 6 in some embodiments is configured to provide a fixed voltage, other embodiments includes a power supply that can produce a variable voltage so as to provide a variable light emittance as light emitted typically is proportional to the voltage applied to the light sources. The power supply 6 is also typically controllable in the sense that power to the light sources can be provided individually to light sources in response to a control signal input to the power supply.

Further, the UV arrangement has a control unit 7 configured to control the power supply 6 to electrically power the light source(s) 4 in response to a power-on signal output from the control unit 7. Alternatively, the control unit 7 may be configured to cut the power to the light source(s) 4 in response to a power-down signal output from the control unit 7.

The control unit 7 is also configured to receive the electrical output from the UV sensor(s) 5.

The control unit 7 may also be configured to carry out one or more, such as all steps in the method aspect of the invention; however, some or all of these steps may be carried out in another unit, such as a computer unit, in which case the control unit 7 forward a sensor output signal to this other unit.

The embodiment disclosed in fig. 1 is disclosed as if each light source 4 is a single element. While this is within the scope of the invention, the each light source 4 may be an ensemble of individual light emitting elements 13. This is shown in figs. 3 and 4.

Thus, an UV arrangement, may have more than two light sources 4, such as three, four, five, six, seven or even eight light sources or even a higher number. As shown in the particular embodiment of figs. 3 and 4, light sources being preferably arranged in a pie-shaped socket structure. In fig. 4, one such pie-shaped socket structure is indicated by the grey shading.

Since one aim of the present invention is to disinfect a fluid, typically water, by radiation of UV light into the fluid, the UV source(s) is each selected to emit light with a wavelength in the UVC band of 200-280 nm, UVA band of 315-400 nm and/or in the UVB band of 280-315 nm. In some preferred embodiments, the UV light is emitted with a wavelength of essentially 265 nm or 245 nm.

The UV source(s) is(are) preferably light emitting diodes (LED),

UV arrangements having a plurality of light sources may either have lights source are configured for emitting light at different wave length or at the same wave length. When UV light sources emitting light a different wave length are applied, the advantage of being able to perform a broader disinfection range may be obtained. It is also worth noting that the above disclosure with respect to Lambert-Beers law is not influenced by the wavelength of the light, and in some embodiments, one wavelength is used during the cycle and another during the operation.

It is sometimes preferred to shield the UV light sources from the fluid, e.g. due to risk of electrical short-cutting by the fluid contacting electrical connections and/or due to avoid mechanical action on the light sources from the fluid itself and/or particles contained in the fluid. Such a shielding is in some embodiments of the UV arrangement provided by the UV source(s) 4 being covered by a UV transparent cover 15. The cover 15 is typically made from glass or a plastic material. It is worth noting that by UV transparent is typically meant that more than 90% such as more than 95% of the UV light is transmitted through the cover 5. One such example on a cover 5 is shown in fig. 3 and will be detailed below.

If may be of use to be able to detect the condition of an UV source, such as detect ageing resulting in less emittance or in general to be able to measure the actual light intensity of the light emitted by an UV source. In such situation, the UV arrangement can further have a further sensor arranged in close proximity to one or more of the UV sources so as to provide a readout essentially representing the light emitted by the UV source(s). By close proximity is typically meant that the sensor is arranged so close to the light source that absorbance of light during its passage from light source to sensor can be ignored. The measurement of light can be a direct measurement, that is light radiates in a straight line between source and sensor or the measurement may be an indirect measurement by which a reflected light is reflected by the sensor.

To prevent or even avoid the fluid to contact the UV sources, UV source(s) 4, and the further sensor (if present) may be encapsulated in fluid tight casing 15 said casing 15 comprising an essentially UV transparent section allowing UV light to be radiated out from the casing 15.

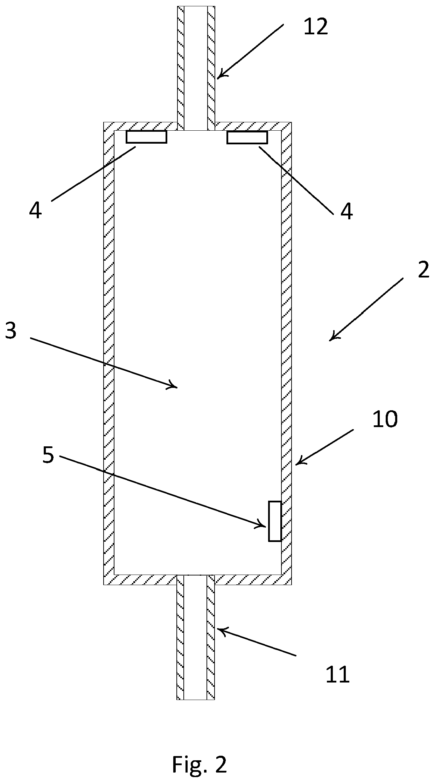

Reference is made to fig. 2 schematically illustrating a reactor 2 adapted to expose a fluid to UV radiation. This detailed description has been focussed on disinfection of water. However, as presented above, an overall purpose can be seen as controlling the quality of the water by measurement and/or disinfection.

The reactor is shown in a cross sectional view. The reactor has a container 2 inside which a void 3 for holding a fluid is present. The void is typically dimensioned in accordance with a predicted usage of water so that the retention time of the fluid in the void 3 is sufficient long to allow for the action of the UV radiation to provide the disinfection. The retention time may be estimated by the volume (m$^3$) of the void divided by volume flow (m$^3$/s).

The reactor also has an UV arrangement of the kind disclosed herein.. As illustrated, the UV sources 4 are arranged at one end of the container so as to expose at least a part of the void 3 to UV radiation. By "at least part of the void" references is made to that the UV sources 4 may not be able to radiate light corners in the void. However, due to flow patterns in the void 3 of the fluid, this does normally not generate an issue, since for instance the flow in the corners will often be characterised as comprising vortices assuring that fluid will eventually be exposed to UV radiation.

In the embodiment shown in fig. 2, an UV sensor 5 is arranged in the opposite end of the container relatively to the position of the light sources so as to receive light emitted from the UV sources 4. As illustrated, the distances from the sensor 5 to each of the light sources 4 are different from each other.

The container 2 has a tubular section 10 inside which said void 3 is present. The tubular part may be cylindrical but the invention is not limited to a cylindrical shape. A first opening 11 is provided at one end of the tubular section 2, which first opening 11 serves as inlet. A second opening 12 is provided at another end of the tubular section 2, which second opening 12 serves as an outlet. Is it noted that the first opening may serve as an outlet and the second opening may serve as an inlet. Thus, the first and the second openings 11, 12 provides passages for fluid to enter and leave said void, and the reactor is typically fluidicly sealed so that fluid can only enter and leave the void, at least during cycle and operation, through the opening 11, 12. The tubular section may have a spherical shape, an ellipsoid shape, may be conical, e.g. shaped as a truncated cone, may have a cross section being triangular, squared, pentagonal, or even polygonal, hexagonal, circular or even ellipsoid.

In a configuration not illustrated, the container 2 has a tubular section 10 inside which said void 3 is present and with the said first and second openings 11, 12 are arranged so that reactor is configured for in-line connection with piping arrangement. By in-line connection is typically meant that the reactor is arranged in piping so that an imaginary piping through the piping continues without major changes in orientation through the reactor.

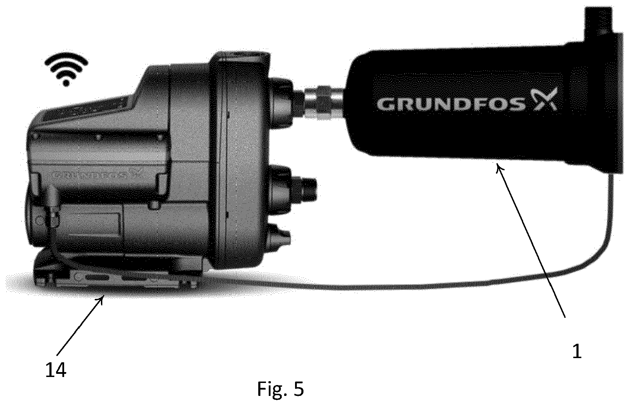

The reactor for disinfection a fluid typically forms part of a system for disinfecting a fluid. Such a system may comprise a pump 14 in fluidic connection with the reactor arranged for pumping fluid through the reactor 1. As shown in fig. 5, such a system may be composed by a pump 14 and a reactor 1 being commonly integrated to form a stand-alone unit.

The system for disinfection a fluid may include a flow switch, such as a sensor, incorporated with the reactor.

Such a flow sensor is configured to provide an electrical output related to fluid flow into/out from said void 3. This electrical output can be used to control whether the light sources are powered or not, e.g. by turning off the light sources in case of no flow through the reactor. In case the flow switch provides a readout corresponding to the actual flow through the reactor, the disinfection can be designed to match a given flow. By knowing the magnitude of the flow the retention time may be estimated and if the retention time is long, the light intensity may be decreased (as longer time is available to expose the fluid to UV radiation) relatively to a situation where the retention time is short often requiring a relatively higher light intensity. This translates into optimisation of the power consumption by the light sources.

The invention provides an elegant way of examining the state of the light sources 4 in a system, where the state of the light source includes ageing of the light source and fouling of the light sources. Both these states results in that less light is emitted than estimated by the nominal light intensity. The steps involved comprises a first measuring cycle during which each light source is sequentially switched on and off, or turning one or more light source sequentially off, and recording the light intensity received by the sensor thereby. This provides what may be labelled a datum measurement for each light source individually. At later point in the first measuring cycle is repeated. By comparing the newly obtained measurement of light with the datum measurement decade, if present, of each of the light sources 4 can be obtained, e.g. by differences in values for each light source individually over time and conclude that decade has occurred if the difference is larger than a pre-set threshold. It is to be noted that this procedure can be continued as long as the reactor or system is in use and that the datum measurement maybe be fixed e.g. as values provided when the reactor or system first time in made up and running.

Another advantage of the present invention is its possibility to determining the absorbance in a fluid exposed to UV radiation in a system or reactor. The method for obtaining the absorbance is based on, such as makes use of, an a priori determined correlation between light intensity $I_0$ emitted by the light source(s) 4, the absorbance $a$ and the light intensity $I_D$ determined by use of the UV sensor 5. The correlation may be the Lambert-Beers law as disclosed above, but it may in general be any correlation, including a table of data, tuned to reflect absorption of light based on $I_0$, $I_D$ and $a$. It may also include the other factors disclosed in the above with reference to Lambert-Beers law.

The method of determining the absorbance includes the step of exposing the fluid to light and determining or estimating the intensity $I_0$ emitted by the light source(s) 4. As will be presented below $I_0$ can be determined in different ways. While exposing the fluid to light with the intensity $I_0$, the light intensity $I_D$ received by the sensor by use of said UV sensor 5 is recorded. Since a correlation exist between emitted light $I_0$, received light $I_D$ and the absorbance $a$, the absorbance is determined by use of the correlation.

The light intensity $I_0$ emitted by the light source(s) 4 is in some embodiments estimated as based on the power consumption of the light source(s) 4. Such a correlation between emitted light and power consumption of the light source is typically available from specification of the light source provided by the manufacturer of the light source and/or can be determined by use of a UV sensor determining emitted light as function of ramping up (or down) the power provided to the light source. The latter has also the advantage of determining emitted time as the light sources ages.

As presented herein, the light emitted by the light sources 4 is emitted at a first wavelength. It is to be noted that in practical implementations a first wavelength is to be interpreted as substantially a single wavelength taking into account what is physical possible to manufacture. The first wave length may be in the UV-A, UV-B or UV-C band. In other embodiments, the light emitted can be composed by light in a single different UV bands or

The correlation between emitted light $I_0$, received light $I_D$ and the absorbance $a$ also includes a fouling factor $k_f$. This fouling factor is designed to take into account during determination of the absorbance $a$ that less light may be received by the UV sensor(s) (5) due to fouling. Such a fouling can occur on the light source and/or on the sensor and results typically from substances present in the water to be disinfection deposit over time on a surface of the light source 4 and/or sensor 5 facing into the void 3 holding the water thereby reducing the transmittance of light out from the light source and/or into the sensor.

As fouling typically is an ongoing mechanism the fouling factor being determined at regular intervals. This may be on hourly, daily or even weekly basis. The invention is not limited to such regular determinations and in some instances the rate of change of fouling is determined over time and in case the rate of change is increasing it may desirable to lower the time between determinations or if the rate of change is decreasing, it may desirable to increase the time between determinations.

The magnitude of the fouling factor correlates to the amount of fouling. With the fouling determined an included in the correlation for determining absorbance it can be determined whether the intensity emitted by the light sources can be controlled to be sufficient high to obtain disinfection or if cleaning is need to remove the fouling.

The fouling factor is typically determined based on obtaining at least two timewise separate measurements of UV light emitted by each of the UV sources (4) by use of the UV sensor. As presented above, this includes an operation where one light source is sequentially switch on and off. The operation may alternative include a procedure where two or more light sources are kept on while one or more are turned off.

Once these measurements are obtained, an absorption coefficient, or a parameter related to the absorption coefficient, for the fluid through which the UV light has travelled is determined, e.g. as disclosed herein. With the absorption coefficient or the related parameter determined a measure for fouling, such as a fouling factor, of the light source(s) (4) and the UV sensor(s) is determined e.g. as disclosed herein

In some specific embodiments, fouling factor is determined on batch of fluid, and the batch of fluid is kept unchanged in volume and composition during the during the determinations.

In order to at least obtain a measure relating the aging of the light sources, the method may further include comparing measurements of UV light from a light source 4 with timewise previous obtained measurement(s) of UV light from the same light source 4. Leaving fouling of such evaluation or taking it into account a decade over time of light will often represent an aging of the light source.

As the sequentially switching on and off of light sources may result in short periods of time where the light intensity is not sufficient to disinfect water as desired, the timewise separate measurements of UV light emitted by the UV source(s) 4 may advantageously be obtained in a cycle where light sources are sequentially switched off and on and the water is not changed in the void during this process.

With the absorbance determined, the intensity of the UV light needed to perform a disinfection may be estimated. Such an estimation is typically based on experimental data providing a correlation between absorbance and needed UV intensity. Further, the mass flow through a reactor may also play a role, since the total amount of UV light exposed to bacteria depends on the retention time of the water. Accordingly, control of the intensity is typically based on both absorbance and mass flow. In case, the intensity needed to disinfect exceed the maximum intensity available by the light sources, then the control scheme may include reducing the mass flow.

List of reference symbols used:

| | |
|---|---|
| 1 | UV reactor |
| 2 | Container |
| 3 | Void for holding a fluid |
| 4 | UV (light) source |
| 5 | UV (light) sensor |
| 6 | Power supply |
| 7 | Control unit |
| 10 | Tubular section |
| 11 | First opening |
| 12 | Second opening |
| 13 | Light emitting element |
| 14 | Pump |
| 15 | Casing |
| 16 | UV transparent cover |

**Claims**

1. A reactor adapted to expose a fluid to UV radiation, the reactor comprising

   • a container (2) having a void (3) for holding a fluid, and
   • an UV arrangement comprising at least two UV sources (4) being configured to emit UV light when electrically powered and at least one UV sensor (5) being configured to provide an electrical output related to received UV intensity when exposed to UV light, said two UV sources (4) and said one UV sensor (5) being positioned relatively to each other so that the distances ($d_1$, $d_2$) from said UV sensor (5) and to each of said UV sources (4) are different from each other so as to provide two different optical path lengths, said two UV source (4) being individually on-off switchable, and

   ◦ the UV sources (4) being arranged in the container to expose at least a part of the void (3) to UV radiation, and
   ◦ the UV sensors (5) being arranged in the container to receive light emitted from the UV sources (4).

2. A reactor according to claim 1, wherein the container (2) has a tubular section (10) inside which said void (3) is present and a first opening (11) at one end of the tubular section (2) and a second opening (12) at another end of the tubular section (2), the first and the second openings (11, 12) provides passages for fluid to enter and leave said void.

3. A reactor according claim 1 or 2, wherein the container (2) has a tubular section (10) inside which said void (3) is present, a first opening (11) and a second opening (12), the first and the second openings (11, 12) provide passages for fluid to enter and leave said void, said first and second openings (11, 12) are arranged so that reactor is configured for in-line connection with a piping arrangement.

4. A system for disinfecting a fluid, said system comprising a pump (14) in fluidic connection with the reactor according to any one of the preceding claims 1-3 for pumping fluid through the reactor (1).

5. A system for disinfection a fluid, said system comprising a flow sensor, such as a flow switch, incorporated with the reactor according to any one of the preceding claims 1-3, said flow sensor being configured to provide an electrical output related to fluid flow into/out from said void (3).

6. A method for examining the state of the light sources (4) in a reactor according to any one of claims 1-3 or the system according to any one of claims 4-5, the method comprising a first measuring cycle comprising sequentially switching each light source on and off, or turning one or more light source sequentially off, and recording the light intensity received by the UV sensor thereby, repeat this first measuring cycle at a later instant and derive from such two measuring cycles decay, if present, of each of the light sources (4).

7. A method for determining absorbance in a fluid exposed to UV radiation in by use of light emitted and sensed in multiple different optical path lengths, the method is based on an a priori determined correlation between light intensity ($I_0$) emitted by UV light source(s) (4), the absorbance ($a$) and the light intensity ($I_D$) determined by use of a UV sensor (5) the method comprising

   • exposing the fluid to light and determining or estimating the intensity ($I_0$) emitted by the light source(s) (4), and
   • recording while exposing the fluid to said light, the light intensity ($I_D$) received by the sensor by use of said UV sensor (5), and
   • determining the absorbance ($a$) based said correlation.

8. A method according to claim 7, wherein the light intensity ($I_0$) emitted by the light source(s) (4) is estimated as based on the power consumption of the light source(s) (4).

9. A method according to any one of claims 6-8, wherein said correlation also includes a fouling factor ($k_f$) to take into account when determining the absorbance ($a$) that less light may be received by the UV sensor(s) (5) due to fouling.

10. A method according to claim 9, wherein said fouling factor being determined at consecutive points in time, such as at regular intervals.

11. A method according to one any of claims 9 or 10, wherein the fouling factor being determined by a method comprising:

   • obtaining at least two timewise separate measurements of UV light emitted by each of the UV sources (4), or by turning one or more light source sequentially off, by use of the UV sensor,
   • determining based on said two timewise separate measurements an absorption coefficient, or a parameter related to the absorption coefficient, for the fluid through which the UV light has travelled, and
   • determining based on the absorption coefficient or the related parameter a measure for fouling, such as a fouling factor, of the light source(s) (4) and the UV sensor(s) (5).

12. A method according to claim 11, wherein the method is carried out on a batch of fluid, and wherein the batch of fluid is kept unchanged in volume and composition during the during the determinations.

13. A method according to any one of the preceding claims 6-12, further comprising comparing measurements of UV light from a light source (4) with previous obtained measurement(s) of UV light from the same light source (4).

14. A method according to any one of the preceding claims 11-13 wherein said timewise separate measurements of UV light emitted by the UV source(s) (4) are obtained in a cycle where said light sources are sequentially switched off and on.

15. A device, such as the reactor according to one of claims 1-3 or the system according to claims 4 or 5, configured to carrying out the method according to any of the of claims 6-14.

Fig. 1

Fig. 2

UV Reactor

LED Lamp

UV LED

SiC photodiode

Fig. 3

Fig. 4

Fig. 5

Europäisches
Patentamt

European
Patent Office

Office européen
des brevets

# EUROPEAN SEARCH REPORT

Application Number

EP 20 20 2041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/090840 A1 (SCHREINER AXEL [DE]) 15 April 2010 (2010-04-15) * paragraphs [0070] - [0077]; figures 6, 7 * | 1-4,6,15 | INV. C02F1/32 A61L2/10 |
| | ----- | | |
| X | US 2003/165398 A1 (WALDO JEFFREY M [US] ET AL) 4 September 2003 (2003-09-04) * paragraphs [0091], [0099], [0136], [0137]; figures 5, 6, 32 * | 1,4-6,15 | |
| | ----- | | |

TECHNICAL FIELDS
SEARCHED (IPC)

C02F
A61L

The present search report has been drawn up for all claims

| Place of search | Date of completion of the search | Examiner |
|---|---|---|
| The Hague | 25 March 2021 | Wolf, Gundula |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

Application Number

EP 20 20 2041

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6(completely); 15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 20 20 2041

The Search Division considers that the present European patent application does not comply with the
requirements of unity of invention and relates to several inventions or groups of inventions, namely:

1. claims: 1-6(completely); 15(partially)

   Reactor adapted to expose a fluid to UV radiation having two
   UV sources and different optical path lengths, whereby the
   UV sources can be switched on/off individually, as well as
   systems and a method using such reactor and a device
   configured to carry out such method
   ---

2. claims: 7-14(completely); 15(partially)

   Method to establish a correlation between the light
   intensity emitted by UV sources and the light intensity
   received by a UV sensor in a fluid and the determination of
   an absorbance based thereon, and a device configured to
   carry out such method
   ---

## ANNEX TO THE EUROPEAN SEARCH REPORT
## ON EUROPEAN PATENT APPLICATION NO.

EP 20 20 2041

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2021

| Patent document cited in search report | Publication date | Patent family member(s) | Publication date |
|---|---|---|---|
| US 2010090840 A1 | 15-04-2010 | DE 102008051239 A1<br>US 2010090840 A1 | 15-04-2010<br>15-04-2010 |
| US 2003165398 A1 | 04-09-2003 | AU 2003275178 A1<br>BR 0314618 A<br>CA 2502415 A1<br>CN 1688344 A<br>CN 101201270 A<br>EP 1551462 A2<br>ES 2392042 T3<br>JP 4632298 B2<br>JP 2006501925 A<br>KR 20050083735 A<br>MX PA05003895 A<br>SG 155051 A1<br>US 2003165398 A1<br>US 2006221329 A1<br>US 2006221330 A1<br>WO 2004033081 A2<br>ZA 200502742 B | 04-05-2004<br>02-08-2005<br>22-04-2004<br>26-10-2005<br>18-06-2008<br>13-07-2005<br>04-12-2012<br>23-02-2011<br>19-01-2006<br>26-08-2005<br>23-11-2005<br>30-09-2009<br>04-09-2003<br>05-10-2006<br>05-10-2006<br>22-04-2004<br>25-04-2007 |

EPO FORM P0459

For more details about this annex : see Official Journal of the European Patent Office, No. 12/82